# EUROPEAN PATENT APPLICATION

(11) **EP 4 850 712 A1**
(43) Date of publication of application: **09.12.2026**
(21) Application number: 25760983.4
(22) Date of filing: 25.02.2025
(51) Int. Cl.: A61M 1/02

(54) **MEDICAL FILTER**

(30) Priority: 29.02.2024 JP 2024029416
(71) Applicant: Terumo Kabushiki Kaisha, Tokyo 151-0072 (JP)
(72) Inventor: KATAOKA, Ryoji, Fujinomiya-shi, Shizuoka 418-0004 (JP)
(74) Representative: TBK
(86) International application number: PCT/JP2025/006367
(87) International publication number: WO 2025/182902

(57) **Abstract**

In a medical filter (10), a housing (12) has a fusion region (32), a non-fusion region (44), and a window portion (46). The non-fusion region (44) is a space different from an internal space (30) of the housing (12). The non-fusion region (44) is a region that is not fused. The non-fusion region (44) is surrounded by the fusion region (32). The window portion (46) is provided in the housing (12) for allowing visual recognition of the non-fusion region (44) from the outside of the housing (12).

## Description

### Technical Field

The present disclosure relates to a medical filter.

### Background Art

JP H07-67952 A discloses a medical filter. The medical filter includes a housing, an inflow port, an outflow port, and a filter medium. The housing is formed by fusing the outer edge portions of two flexible sheets. The inflow port and the outflow port are respectively fused to a peripheral edge portion of the housing. The filter medium is fused to the housing and disposed in an internal space of the housing. The filter medium separates the internal space of the housing into an inflow region and an outflow region. Liquid (blood) flows into the inflow region through the inflow port. The filter medium blocks passage of a predetermined component (white blood cells) of liquid flowing into the inflow region. The liquid having passed through the filter medium flows into the outflow region. The liquid flowing into the outflow region flows out of the housing through the outflow port.

### Citation List

### Patent Literature

Patent Literature 1: JP H07-67952 A

### Summary of Invention

### Technical Problem

It is desired to detect an abnormality of fusion (fusion abnormality) in a medical filter at an early stage.

An object of the present invention is to solve the above-described problem.

(1) An aspect of the present invention is a medical filter including: a housing formed by fusing an outer edge portion of a first sheet portion and an outer edge portion of a second sheet portion; an inflow port that is fused to a peripheral edge portion of the housing and allows liquid to flow into an internal space of the housing from an outside of the housing; a filter medium that is fused to the housing and is disposed in the internal space; and an outflow port that is fused to the peripheral edge portion and allows the liquid having passed through the filter medium to flow out to the outside, in which the filter medium separates the internal space into an inflow region into which the liquid flows through the inflow port and an outflow region from which the liquid having passed through the filter medium flows out to the outside through the outflow port, and the housing includes a non-fusion region that is a space different from the internal space, is a region that is not fused and is surrounded by a fusion region; and a window portion for allowing visual recognition of the non-fusion region from the outside.

According to this configuration, when the liquid is introduced into the internal space of the housing, in a case where the liquid flows into the non-fusion region, a fusion abnormality occurs inside the medical filter. Thus, by visually recognizing inflow of the liquid in the non-fusion region from the outside of the housing through the window portion, it is possible to detect an abnormality of the medical filter at an early stage. In addition, it is possible to reduce loss of the liquid and suppress leakage of the liquid to the outside of the medical filter by detecting an abnormality of the medical filter at an early stage.

(2) In the medical filter according to (1), the filter medium may be formed in a bag shape, an inside of the filter medium may be the inflow region, the fusion region may include a peripheral edge fusion region that fuses the peripheral edge portion and a filter medium fusion region that is fused to the filter medium, and the filter medium fusion region may separate an inside of the housing into the internal space and the non-fusion region.

The filter medium fusion region is fused to the filter medium, and thus, bonding force by fusion tends to be lower than that of the peripheral edge fusion region which is the fused portion between the first sheet portion and the second sheet portion. If a fusion abnormality occurs in the filter medium fusion region, the liquid flows into the non-fusion region. This is effective for early detection of an abnormality of the medical filter.

(3) In the medical filter according to (2), the inflow port may be inserted into the inflow region through a proximal portion of the filter medium, the filter medium fusion region may be integrally fused to the proximal portion of the filter medium and the inflow port, the non-fusion region may be formed between the filter medium fusion region and the peripheral edge fusion region, a proximal end of the filter medium may be located in the non-fusion region, and the window portion may be provided in the housing so as to face the non-fusion region.

The three members of the housing, the filter medium, and the inflow port are fused, and thus, bonding force by fusion is likely to be lower in a fused portion among the filter medium fusion region, the proximal portion of the filter medium, and the inflow port than in the peripheral edge fusion region. If a fusion abnormality occurs in the fused portion, the liquid flows into the non-fusion region via the proximal end of the filter medium. The proximal end of the filter medium is located in the non-fusion region beyond the filter medium fusion region. In addition, the window portion faces the non-fusion region. Thus, a user can visually recognize the proximal end of the filter medium located in the non-fusion region through the window portion. In addition, the user can easily confirm whether the liquid flows into the non-fusion region through the window portion. This is effective for early detection of an abnormality of the medical filter.

(4) In the medical filter according to (3), the non-fusion region may extend along a width direction of the housing orthogonal to a thickness direction of the housing and an axial direction of the inflow port, and a width of the non-fusion region in the width direction may be larger than a width of the filter medium in the width direction.

According to this configuration, the window portion facing the non-fusion region can be enlarged. This makes it easier to view the non-fusion region from the outside of the housing through the window portion.

(5) In the medical filter according to (4), a width of the outflow region in the width direction may be larger than the width of the filter medium in the width direction, and the filter medium fusion region may separate the non-fusion region and the outflow region.

If a fusion abnormality occurs in the filter medium fusion region, the liquid flows into the non-fusion region from the outflow region. This is more effective for early detection of an abnormality of the medical filter.

(6) In the medical filter according to (2), the inflow port is inserted into the inflow region through a proximal portion of the filter medium, the filter medium fusion region may be integrally fused to at least one of two side edge portions of the filter medium between the proximal portion and a distal portion of the filter medium, the proximal portion, and the inflow port, the non-fusion region may include a width direction portion formed between the filter medium fusion region and the peripheral edge fusion region and extending along a width direction of the housing orthogonal to a thickness direction of the housing and an axial direction of the inflow port, and an axial direction portion extending along the axial direction, a proximal end of the filter medium may be located in the width direction portion, a side edge of at least one of the side edge portions may be located in the axial direction portion, and the window portion may be provided in the housing so as to face the non-fusion region.

Three members of the housing, the filter medium, and the inflow port are fused, and thus, bonding force by fusion tends to be lower in a fused portion among the filter medium fusion region, the proximal portion of the filter medium, and the inflow port than in the peripheral edge fusion region which is a fused portion between the first sheet portion and the second sheet portion. In addition, the filter medium fusion region is fused to the filter medium, and thus, bonding force by fusion between the filter medium fusion region and the proximal portion and the side edge portion of the filter medium tends to be lower than that in the peripheral edge fusion region. If a fusion abnormality occurs in these fused portions, the liquid flows into the non-fusion region via the proximal end and the side edge portion of the filter medium. The proximal end and the side edge portion of the filter medium are located in the non-fusion region beyond the filter medium fusion region. In addition, the window portion faces the non-fusion region. Thus, the user can visually recognize the proximal end and the side edge portion of the filter medium located in the non-fusion region through the window portion. In addition, the user can easily confirm whether the liquid flows into the non-fusion region through the window portion. This is effective for early detection of an abnormality of the medical filter.

(7) In the medical filter according to (6), a width of the width direction portion in the width direction may be larger than a width of the filter medium in the width direction.

A portion of the window portion facing the width direction portion can be enlarged. This makes it easier to view the width direction portion from the outside of the housing through the window portion.

(8) In the medical filter according to (7), the axial direction portion may extend toward the outflow port from the distal portion, the outflow region may extend toward the outflow port from the distal portion, and the filter medium fusion region may separate the non-fusion region and the outflow region.

If a fusion abnormality occurs in the filter medium fusion region, the liquid flows into the non-fusion region from the outflow region. This is more effective for early detection of an abnormality of the medical filter. In addition, a portion of the window portion facing the axial direction portion can be enlarged. This makes it easier to view the axial direction portion from the outside of the housing through the window portion.

According to the present invention, when the liquid is introduced into the internal space of the housing, in a case where the liquid flows into the non-fusion region, a fusion abnormality occurs inside the medical filter. Thus, by visually recognizing inflow of the liquid in the non-fusion region from the outside of the housing through the window portion, it is possible to detect an abnormality of the medical filter at an early stage. In addition, it is possible to reduce loss of the liquid and suppress leakage of the liquid to the outside of the medical filter by detecting an abnormality of the medical filter at an early stage.

### Brief Description of Drawings

[Fig. 1] Fig. 1 is a front view of a medical filter according to a first embodiment.
[Fig. 2] Fig. 2 is a cross-sectional view taken along a line II-II of Fig. 1.
[Fig. 3] Fig. 3 is a cross-sectional view taken along a line III-III of Fig. 1.
[Fig. 4] Figs. 4A and 4B are partial cross-sectional views illustrating another configuration example of a filter medium.
[Fig. 5] Fig. 5 is a front view of a medical filter according to a second embodiment.
[Fig. 6] Fig. 6 is a cross-sectional view taken along a line VI-VI of Fig. 5.
[Fig. 7] Fig. 7 is a front view of a medical filter according to a third embodiment.

### Description of Embodiments

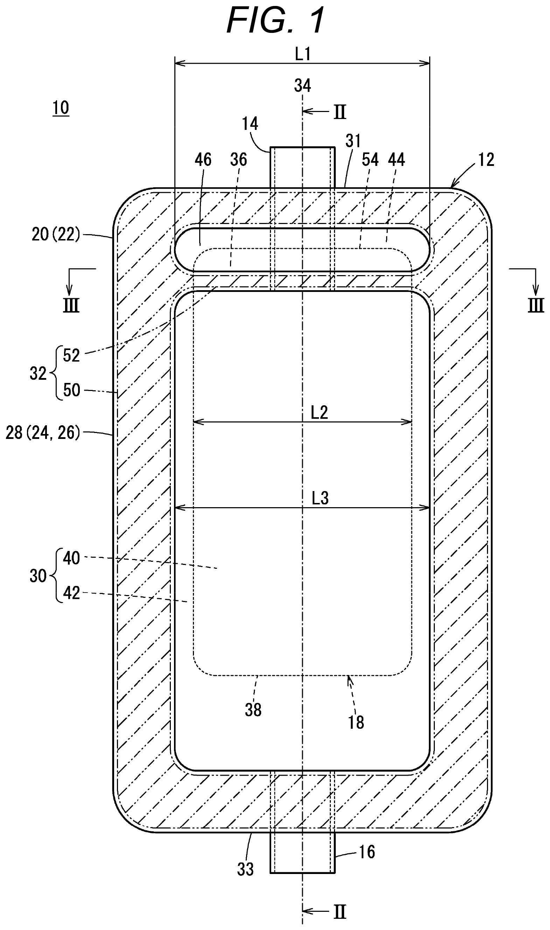

A medical filter 10 according to a first embodiment will be described with reference to Figs. 1 to 3. The medical filter 10 is a filter for filtering liquid. The medical filter 10 is a filter for suppressing passage of a predetermined component in liquid. The medical filter 10 can be a filter for blocking passage of a predetermined component (for example, white blood cells) in blood.

The medical filter 10 includes a housing 12, an inflow port 14, an outflow port 16, and a filter medium 18.

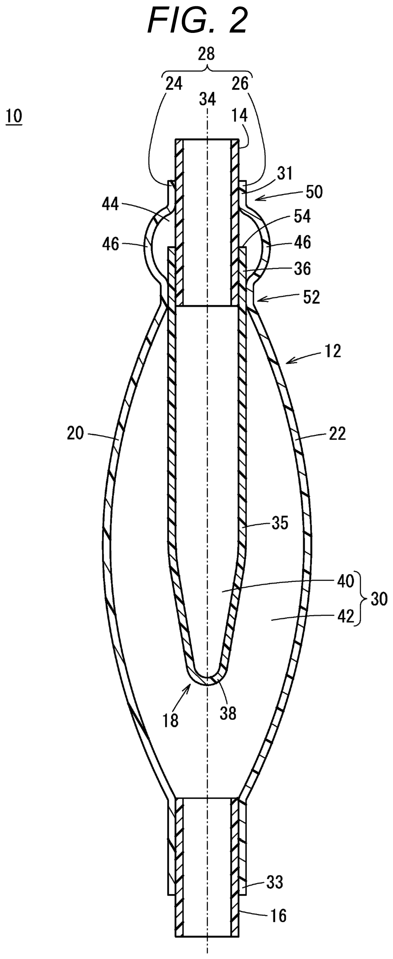

The housing 12 is a rectangular bag body. The housing 12 includes a first sheet portion 20 and a second sheet portion 22. The first sheet portion 20 and the second sheet portion 22 are two separate flexible sheets. The first sheet portion 20 and the second sheet portion 22 are sheets having transparency. As illustrated in Figs. 1 and 2, the first sheet portion 20 and the second sheet portion 22 are rectangular sheets having the same size and thickness. A constituent material of the first sheet portion 20 and the second sheet portion 22 is synthetic resin. Specific examples of the constituent material of the first sheet portion 20 and the second sheet portion 22 include thermoplastic elastomers such as soft vinyl chloride, polyurethane, an ethylene-vinyl acetate copolymer, a hydrogenated product of a styrene-butadienestyrene copolymer, a styrene-isoprene-styrene copolymer or a hydrogenated product thereof, and mixtures of thermoplastic elastomers and softeners such as polyolefins and ethylene-ethyl acrylate.

The housing 12 is formed in a bag shape by superimposing the first sheet portion 20 and the second sheet portion 22 and fusing an outer edge portion 24 of the first sheet portion 20 and an outer edge portion 26 of the second sheet portion 22. The fusion of the first sheet portion 20 and the second sheet portion 22 may be external heating fusion by heat sealing. Alternatively, the fusion of the first sheet portion 20 and the second sheet portion 22 may be internal fusion by a high-frequency welder, an ultrasonic welder, or the like. In the following description, "fusion" includes the above-described external heating fusion or internal fusion.

The fused outer edge portions 24 and 26 of the first sheet portion 20 and second sheet portion 22 form a peripheral edge portion 28 of the housing 12. Further, by fusing the outer edge portions 24 and 26 of the first sheet portion 20 and the second sheet portion 22, an internal space 30 is formed inside the housing 12. In the following description, the fused portion of the housing 12 is referred to as a fusion region 32. In Fig. 1, the fusion region 32 is illustrated by a two-dot chain line.

Note that the housing 12 may be formed by folding and superimposing one flexible sheet and fusing an outer edge portion of the flexible sheet. In this case, one portion of the folded flexible sheet becomes the first sheet portion 20, and the other portion becomes the second sheet portion 22.

As illustrated in Figs. 1 and 2, the inflow port 14 is a tubular member. The inflow port 14 is provided at a first end portion 31 which is one end portion of the housing 12. Specifically, the inflow port 14 penetrates the first end portion 31 of the peripheral edge portion 28 of the housing 12 and is inserted into the internal space 30 of the housing 12. The inflow port 14 is fused to the first end portion 31 of the housing 12. A constituent material of the inflow port 14 is, for example, synthetic resin. The inflow port 14 allows liquid such as blood to flow into the internal space 30 of the housing 12 from the outside of the housing 12.

The outflow port 16 is a tubular member having the same size and shape as the size and shape of the inflow port 14. The outflow port 16 is provided at a second end portion 33 that is the other end portion of the housing 12. Specifically, the outflow port 16 penetrates the second end portion 33 of the peripheral edge portion 28 of the housing 12 and is inserted into the internal space 30 of the housing 12. The outflow port 16 is fused to the second end portion 33 of the housing 12. A constituent material of the outflow port 16 is the same as the constituent material of the inflow port 14. The outflow port 16 allows liquid in the internal space 30 of the housing 12 to flow out of the housing 12.

The inflow port 14 and the outflow port 16 are fused to the housing 12 while facing each other. In other words, the inflow port 14 and the outflow port 16 are coaxially fused to the housing 12. The inflow port 14 and the outflow port 16 are disposed on a central axis 34 of the inflow port 14.

The filter medium 18 is disposed in the internal space 30 of the housing 12. The filter medium 18 is formed in a rectangular shape in a front view of Fig. 1. The filter medium 18 is formed in a bag shape in the cross-sectional view of Fig. 2. The filter medium 18 is fused to the housing 12. As illustrated in Fig. 1, the filter medium 18 is fused to the housing 12 coaxially with the central axis 34. A proximal portion 36 of the filter medium 18 faces the inflow port 14. A distal portion 38 of the filter medium 18 faces the outflow port 16.

The filter medium 18 is a porous body of synthetic resin or synthetic fiber. Examples of the constituent material of the filter medium 18 include polyester, polyamide, polypropylene, polyethylene, polyurethane, polyvinyl chloride, acrylonitrile, and styrene-based elastomer.

The filter medium 18 is formed by folding one rectangular flexible sheet 35 made of the above-described constituent material into a U shape and fusing an outer edge portion of the folded flexible sheet 35. Thus, the filter medium 18 is formed in a bag shape by fusing the outer edge portion of one flexible sheet 35. Alternatively, the filter medium 18 may be formed in a bag shape by superimposing two flexible sheets 35 made of the above-described constituent material and fusing outer edge portions of the two flexible sheets 35.

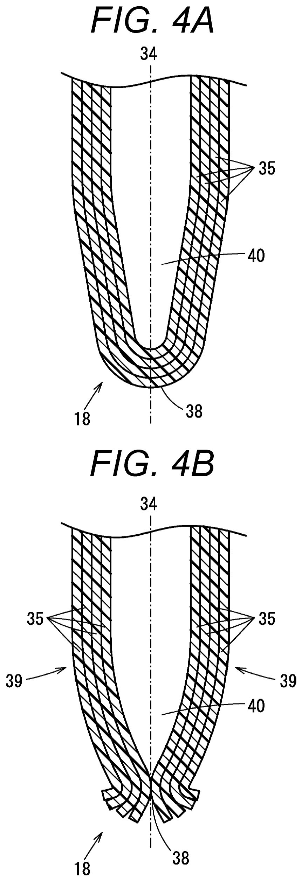

The filter medium 18 may be formed as illustrated in Figs. 4A and 4B.

The filter medium 18 illustrated in Fig. 4A is formed by superimposing a plurality of rectangular flexible sheets 35, folding them into a U shape, and fusing the outer edge portions of the plurality of folded flexible sheets 35.

The filter medium 18 illustrated in Fig. 4B is formed in a bag shape by superimposing a plurality of rectangular flexible sheets 35 and fusing outer edge portions of the plurality of flexible sheets 35. Specifically, one sheet body 39 in which a plurality of flexible sheets 35 is superimposed and the other sheet body 39 in which a plurality of flexible sheets 35 is superimposed are prepared. An outer edge portion of the one sheet body 39 and an outer edge portion of the other sheet body 39 are fused to form the filter medium 18 in a bag shape. In this case, the outer edge portion of the one sheet body 39 is an outer edge portion of the plurality of flexible sheets 35 constituting the one sheet body 39. The outer edge portion of the other sheet body 39 is an outer edge portion of the plurality of flexible sheets 35 constituting the other sheet body 39.

As illustrated in Figs. 1 and 2, the inflow port 14 passes through the proximal portion 36 of the bag-shaped filter medium 18. In other words, the inflow port 14 communicates with the inside of the filter medium 18. The housing 12, the proximal portion 36 of the filter medium 18, and the inflow port 14 are integrally fused.

The filter medium 18 separates the internal space 30 of the housing 12 into an inflow region 40 and an outflow region 42. Specifically, the inflow region 40 is a space inside the filter medium 18. The inflow region 40 communicates with the outside of the housing 12 via the inflow port 14. Liquid flows into the inflow region 40 through the inflow port 14.

The filter medium 18 suppresses passage of a predetermined component (for example, white blood cells in blood) in the liquid flowing into the inflow region 40. The filter medium 18 allows passage of components other than the predetermined component in the liquid.

The outflow region 42 is a space outside the filter medium 18 in the internal space 30 of the housing 12. The outflow region 42 communicates with the outflow port 16. The liquid having passed through the filter medium 18 flows into the outflow region 42. The liquid flowing into the outflow region 42 flows out of the housing 12 through the outflow port 16.

The housing 12 further includes a fusion region 32, a non-fusion region 44, and a window portion 46.

As illustrated in Figs. 1 to 3, the non-fusion region 44 is a space formed inside the housing 12. The non-fusion region 44 is a space different from the internal space 30 of the housing 12. The non-fusion region 44 is a space region that is not fused. The non-fusion region 44 is surrounded by the fusion region 32.

The fusion region 32 includes a peripheral edge fusion region 50 and a filter medium fusion region 52.

The peripheral edge fusion region 50 is a fused portion between the outer edge portion 24 of the first sheet portion 20 and the outer edge portion 26 of the second sheet portion 22 in the peripheral edge portion 28 of the housing 12. In the peripheral edge fusion region 50, the outer edge portion 24 of the first sheet portion 20 and the outer edge portion 26 of the second sheet portion 22 are directly fused.

The peripheral edge fusion region 50 is integrally fused with the inflow port 14 and the outflow port 16.

The inflow port 14 is integrally fused to the housing 12 in a state of being sandwiched between the peripheral edge fusion region 50 and the filter medium fusion region 52. Specifically, a portion of the inflow port 14 located in the non-fusion region 44 is fused only to the proximal end 54 of the filter medium 18. A portion of the inflow port 14 located in the non-fusion region 44 is not fused to the housing 12.

An opening of the inflow port 14 on the outflow port 16 side (an opening communicating with the inflow region 40) is open at a position of the filter medium fusion region 52. Alternatively, the opening of the inflow port 14 on the outflow port 16 side may be open at a position closer to the outflow port 16 than the filter medium fusion region 52.

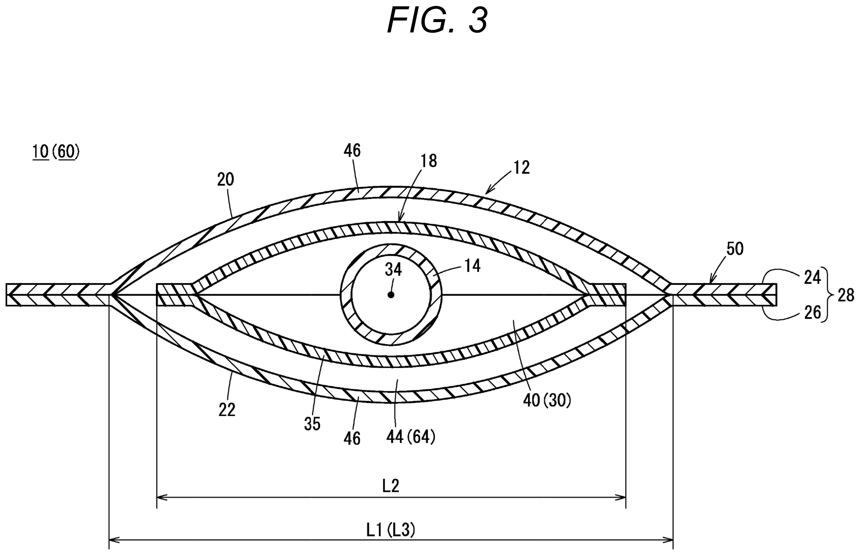

The filter medium fusion region 52 is a fused portion of the housing 12 with the filter medium 18. The filter medium fusion region 52 extends along the width direction (the left-right direction in Figs. 1 and 3) of the housing 12 orthogonal to the axial direction of the inflow port 14 and the thickness direction of the housing 12. The filter medium fusion region 52 is formed inside the peripheral edge fusion region 50 in the width direction of the housing 12.

As illustrated in Fig. 1, both ends of the filter medium fusion region 52 in the width direction are connected to the peripheral edge fusion region 50. As illustrated in Figs. 1 and 2, the filter medium fusion region 52 separates the inside of the housing 12 into the internal space 30 and the non-fusion region 44. Specifically, the filter medium fusion region 52 separates the non-fusion region 44 and the outflow region 42 of the internal space 30. The filter medium fusion region 52 is integrally fused to a proximal portion 36 of the filter medium 18 and the inflow port 14. Although schematically illustrated in Fig. 2, strictly speaking, in the filter medium fusion region 52, the filter medium 18 is impregnated with part of the housing 12 (the first sheet portion 20 and the second sheet portion 22). In the filter medium fusion region 52, an outer peripheral surface of the inflow port 14 is surrounded by the filter medium 18 and is surrounded by the housing 12. Thus, in a portion where the inflow port 14 is located in the filter medium fusion region 52, the housing 12, the filter medium 18, and the inflow port 14 are integrally fused by the filter medium 18 being impregnated with part of the housing 12. In the filter medium fusion region 52, the first sheet portion 20, the filter medium 18, and the second sheet portion 22 are integrally fused by the filter medium 18 being impregnated with part of the housing 12 in a portion other than a portion where the inflow port 14 is located.

The non-fusion region 44 is formed between the filter medium fusion region 52 and a fused portion of the peripheral edge fusion region 50 with the inflow port 14. The proximal end 54 of the filter medium 18 is located in the non-fusion region 44. Specifically, the proximal end 54 of the filter medium 18 is located in the non-fusion region 44 beyond the filter medium fusion region 52.

As illustrated in Figs. 1 and 3, the non-fusion region 44 extends along a width direction of the housing 12. A width L1, which is a dimension of the non-fusion region 44 in the width direction, is larger than a width L2, which is a dimension of the filter medium 18 in the width direction (L1 > L2). A width L3, which is a dimension of the outflow region 42 in the width direction, is larger than the width L2, which is a dimension of the filter medium 18 in the width direction (L3 > L2). The outflow region 42 extends toward the outflow port 16 from the distal portion 38 of the filter medium 18.

As illustrated in Figs. 1 to 3, the window portion 46 is part of the housing 12. The window portion 46 is part of the first sheet portion 20 and the second sheet portion 22 constituting the housing 12. The window portion 46 is a window portion for allowing visual recognition of the non-fusion region 44 from the outside of the housing 12. The window portion 46 is provided in the housing 12 so as to face the non-fusion region 44. The window portion 46 is provided between the non-fusion region 44 and the peripheral edge fusion region 50 in the housing 12 so as to face the non-fusion region 44. Thus, in the front view of Fig. 1, a size of the window portion 46 is the same as a size of the non-fusion region 44. Thus, a user can visually recognize the proximal end 54 of the filter medium 18 located in the non-fusion region 44 through the window portion 46. As illustrated in Figs. 2 and 3, the window portion 46 is provided in each of the first sheet portion 20 and the second sheet portion 22 so as to face the non-fusion region 44.

The window portion 46 is only required to be formed in at least one of the first sheet portion 20 or the second sheet portion 22.

The medical filter 10 according to the first embodiment is used as follows.

Liquid such as blood flows into the inflow region 40 from the outside of the housing 12 via the inflow port 14. The filter medium 18 blocks passage of a predetermined component such as white blood cells for the liquid flowing into the inflow region 40 and allows components other than the predetermined component to pass through. The liquid having passed through the filter medium 18 flows into the outflow region 42. The liquid flowing into the outflow region 42 flows out of the housing 12 through the outflow port 16.

In this event, there is a possibility that the liquid passes through a portion having low bonding force by fusion in the fusion region 32. Such a fusion abnormality is likely to occur in the fused portion between the filter medium fusion region 52 and the filter medium 18 and in the fused portion among the filter medium fusion region 52, the proximal portion 36 of the filter medium 18, and the inflow port 14. In other words, the bonding force by fusion is lower in the fused portion between the filter medium fusion region 52 and the filter medium 18 than in the peripheral edge fusion region 50. In addition, three members of the filter medium fusion region 52, the filter medium 18, and the inflow port 14 are fused, and thus, the bonding force by fusion tends to be lower than that in the peripheral edge fusion region 50.

Thus, if a fusion abnormality occurs, the liquid passes through the filter medium fusion region 52 and flows into the non-fusion region 44. The user can confirm whether the liquid flows into the non-fusion region 44 by visually recognizing the non-fusion region 44 through the window portion 46. In a case where it is confirmed that the liquid flows into the non-fusion region 44, the user can easily recognize that an abnormality occurs in the medical filter 10.

The first embodiment has the following effects.

As illustrated in Figs. 1 to 3, when the liquid is introduced into the internal space 30 of the housing 12, in a case where the liquid flows into the non-fusion region 44, a fusion abnormality occurs inside the medical filter 10. It is therefore possible to detect an abnormality of the medical filter 10 at an early stage by visually recognizing the inflow of the liquid in the non-fusion region 44 from the outside of the housing 12 through the window portion 46. In addition, early detection of an abnormality of the medical filter 10 makes it possible to reduce loss of the liquid and suppress leakage of the liquid to the outside of the medical filter 10.

The filter medium fusion region 52 is fused to the filter medium 18, and thus, the bonding force by fusion tends to be lower than that of the peripheral edge fusion region 50 which is a fused portion between the first sheet portion 20 and the second sheet portion 22. If a fusion abnormality occurs in the filter medium fusion region 52, the liquid flows into the non-fusion region 44. This is effective for early detection of an abnormality of the medical filter 10.

The three members of the housing 12, the filter medium 18, and the inflow port 14 are fused, and thus, the bonding force by fusion tends to be lower in the fused portion among the filter medium fusion region 52, the proximal portion 36 of the filter medium 18, and the inflow port 14 than in the peripheral edge fusion region 50. If a fusion abnormality occurs in the fused portion, the liquid flows into the non-fusion region 44 via the proximal end 54 of the filter medium 18. The proximal end 54 of the filter medium 18 is located in the non-fusion region 44 beyond the filter medium fusion region 52. In addition, the window portion 46 faces the non-fusion region 44. Thus, the user can visually recognize the proximal end 54 of the filter medium 18 located in the non-fusion region 44 through the window portion 46. In addition, the user can easily confirm whether the liquid flows into the non-fusion region 44 through the window portion 46. This is effective for early detection of an abnormality of the medical filter 10.

The non-fusion region 44 extends along the width direction of the housing 12, and the width L1 of the non-fusion region 44 in the width direction is larger than the width L2 of the filter medium 18 in the width direction (L1 > L2). This can enlarge the window portion 46 facing the non-fusion region 44. This results in making it easier to view the non-fusion region 44 from the outside of the housing 12 through the window portion 46.

The width L3 of the outflow region 42 in the width direction is larger than the width L2 of the filter medium 18 in the width direction (L3 > L2). In addition, the filter medium fusion region 52 separates the non-fusion region 44 and the outflow region 42. If a fusion abnormality occurs in the filter medium fusion region 52, the liquid flows into the non-fusion region 44 from the outflow region 42. This is more effective for early detection of an abnormality of the medical filter 10.

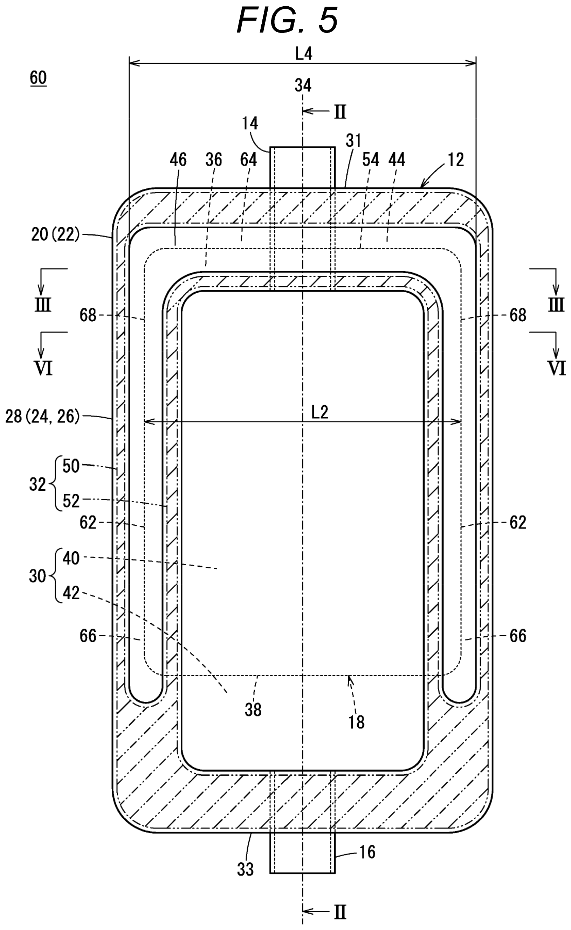

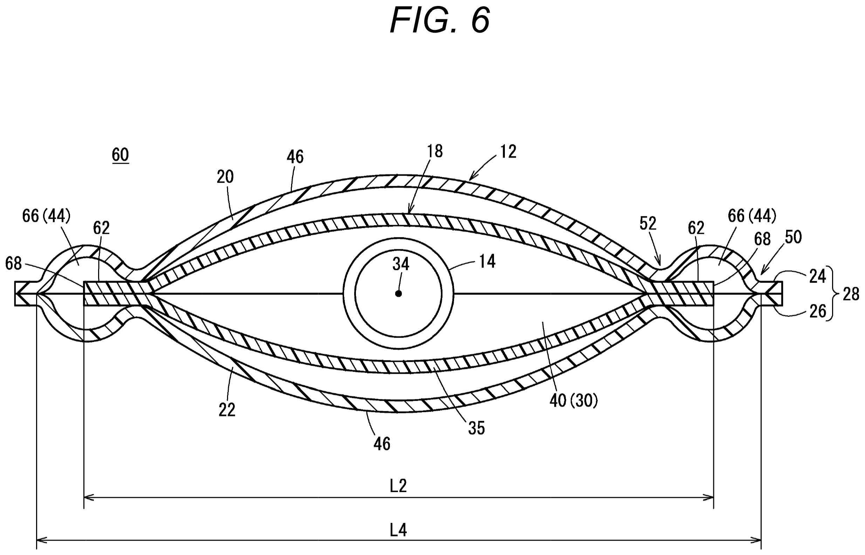

A medical filter 60 according to a second embodiment will be described with reference to Figs. 5 and 6. In the medical filter 60 according to the second embodiment, the same components as those of the medical filter 10 according to the first embodiment (see Figs. 1 to 4B) are denoted by the same reference numerals, and detailed description thereof will be omitted.

In the medical filter 60 according to the second embodiment, the filter medium fusion region 52 is integrally fused to two side edge portions 62 between the proximal portion 36 and the distal portion 38 of the filter medium 18, the proximal portion 36 of the filter medium 18, and the inflow port 14. Thus, as illustrated in Fig. 5, the filter medium fusion region 52 is formed in a U shape. Note that the filter medium fusion region 52 may be integrally fused to at least one side edge portion 62 of the two side edge portions 62 of the filter medium 18, the proximal portion 36 of the filter medium 18, and the inflow port 14.

In the second embodiment, the non-fusion region 44 is formed in a U shape between the filter medium fusion region 52 and the peripheral edge fusion region 50. The non-fusion region 44 has a width direction portion 64 and two axial direction portions 66.

The width direction portion 64 is formed to face the proximal portion 36 of the filter medium 18. The width direction portion 64 extends along the width direction. The proximal end 54 of the filter medium 18 is located in the width direction portion 64. In other words, the proximal end 54 of the filter medium 18 is located in the width direction portion 64 beyond the filter medium fusion region 52.

Each of the two axial direction portions 66 is formed to face the side edge portion 62 of the filter medium 18. Each of the two axial direction portions 66 extends along the axial direction. Side edges 68 of the filter medium 18 are located in the axial direction portions 66. In other words, the side edge portions 62 of the filter medium 18 are located in the axial direction portions 66 beyond the filter medium fusion region 52.

The window portion 46 is provided in the housing 12 so as to face the non-fusion region 44. Thus, the window portion 46 is formed in a U shape. The user can visually recognize the proximal end 54 and the two side edge portions 62 of the filter medium 18 located in the non-fusion region 44 through the window portion 46. A width L4, which is a dimension of the width direction portion 64 in the width direction, is larger than the width L2 of the filter medium 18 in the width direction (L4 > L2). The axial direction portions 66 extend toward the outflow port 16 from the distal portion 38 of the filter medium 18.

Note that the width direction portion 64 and the two axial direction portions 66 may be divided. In addition, in a case where the filter medium fusion region 52 is integrally fused to one side edge portion 62 of the two side edge portions 62 of the filter medium 18, the proximal portion 36 of the filter medium 18, and the inflow port 14, the filter medium fusion region 52 may be formed in an L shape. In this case, the non-fusion region 44 and the window portion 46 are formed in an L shape so as to correspond to the filter medium fusion region 52.

The second embodiment has the following effects.

As illustrated in Figs. 5 and 6, three members of the housing 12, the filter medium 18, and the inflow port 14 are fused, and thus, bonding force by fusion is likely to be lower in the fused portion among the filter medium fusion region 52, the proximal portion 36 of the filter medium 18, and the inflow port 14 than in the peripheral edge fusion region 50 which is the fused portion between the first sheet portion 20 and the second sheet portion 22. In addition, the bonding force by fusion tends to be lower in the fused portion between the filter medium fusion region 52 and the proximal portion 36 and the side edge portion 62 of the filter medium 18 than in the peripheral edge fusion region 50. If a fusion abnormality occurs in these fused portions, the liquid flows into the non-fusion region 44 via the proximal end 54 and the side edge portion 62 of the filter medium 18. The proximal end 54 and the side edge portions 62 of the filter medium 18 are located in the non-fusion region 44 beyond the filter medium fusion region 52. In other words, the proximal end 54 and the side edge portions 62 of the filter medium 18 are not fused to the housing 12. The proximal end 54 and the side edge portions 62 of the filter medium 18 are free ends located in the non-fusion region 44. In addition, the window portion 46 faces the non-fusion region 44. Thus, the user can visually recognize the proximal end 54 and the side edge portions 62 of the filter medium 18 located in the non-fusion region 44 through the window portion 46. In addition, the user can easily confirm whether the liquid flows into the non-fusion region 44 through the window portion 46. This is effective for early detection of an abnormality of the medical filter 60.

The width L4 of the width direction portion 64 in the width direction is larger than the width L2 of the filter medium 18 in the width direction (L4 > L2). This can enlarge a portion of the window portion 46 facing the width direction portion 64. This results in making it easier to view the width direction portion 64 from the outside of the housing 12 through the window portion 46.

If a fusion abnormality occurs in the filter medium fusion region 52, the liquid flows into the non-fusion region 44 from the outflow region 42. This is more effective for early detection of an abnormality of the medical filter 60. Further, this can enlarge a portion of the window portion 46 facing the axial direction portions 66. This makes it easier to view the axial portion 66 from the outside of the housing 12 through the window portion 46.

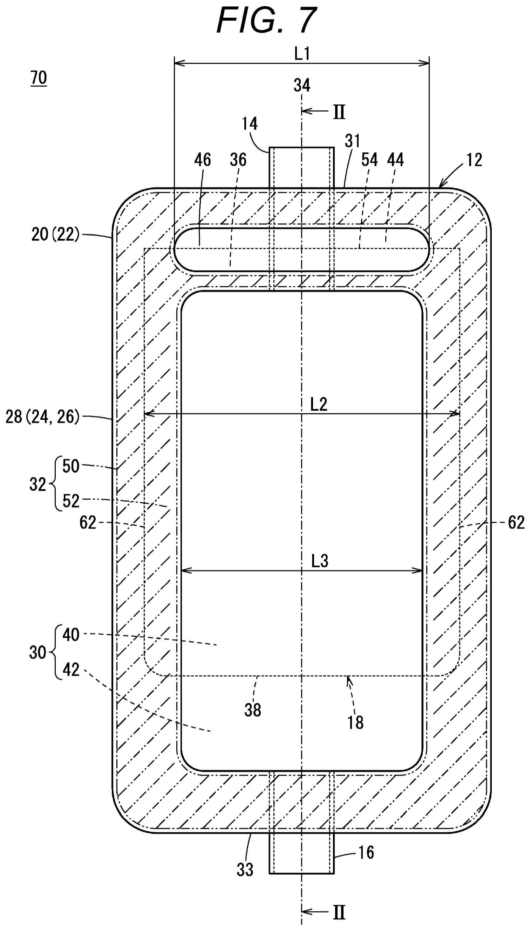

A medical filter 70 according to a third embodiment will be described with reference to Fig. 7.

In the medical filter 70 according to the third embodiment, the filter medium fusion region 52 is integrally fused to the proximal portion 36 and the two side edge portions 62 of the filter medium 18 and the inflow port 14. Thus, the filter medium fusion region 52 is formed in a U shape. As illustrated in Fig. 7, the width L2 of the filter medium 18 is larger than the width L1 of the non-fusion region 44 (L2 > L1). Note that the filter medium fusion region 52 may be integrally fused to at least one side edge portion 62 of the two side edge portions 62 of the filter medium 18, the proximal portion 36 of the filter medium 18, and the inflow port 14.

Also in the third embodiment, the same effects as those of the first embodiment can be obtained.

Although the present disclosure has been described in detail, the present disclosure is not limited to the individual embodiments described above. Various additions, substitutions, modifications, partial deletions, and the like, may be made to these embodiments without departing from the gist of the present disclosure or without departing from the spirit of the present disclosure derived from the content described in the claims and equivalents thereof.

## Claims

1. A medical filter comprising:
a housing formed by fusing an outer edge portion of a first sheet portion and an outer edge portion of a second sheet portion;
an inflow port that is fused to a peripheral edge portion of the housing and allows liquid to flow into an internal space of the housing from an outside of the housing;
a filter medium that is fused to the housing and is disposed in the internal space; and
an outflow port that is fused to the peripheral edge portion and allows the liquid having passed through the filter medium to flow out to the outside,
wherein
the filter medium separates the internal space into an inflow region into which the liquid flows through the inflow port and an outflow region from which the liquid having passed through the filter medium flows out to the outside through the outflow port, and
the housing includes:
a non-fusion region that is a space different from the internal space, is a region that is not fused and is surrounded by a fusion region, and
a window portion for allowing visual recognition of the non-fusion region from the outside.

2. The medical filter according to claim 1, wherein
the filter medium is formed in a bag shape,
an inside of the filter medium is the inflow region,
the fusion region includes a peripheral edge fusion region that fuses the peripheral edge portion and a filter medium fusion region that is fused to the filter medium, and
the filter medium fusion region separates an inside of the housing into the internal space and the non-fusion region.

3. The medical filter according to claim 2, wherein
the inflow port is inserted into the inflow region through a proximal portion of the filter medium,
the filter medium fusion region is integrally fused to the proximal portion of the filter medium and the inflow port,
the non-fusion region is formed between the filter medium fusion region and the peripheral edge fusion region,
a proximal end of the filter medium is located in the non-fusion region, and
the window portion is provided in the housing so as to face the non-fusion region.

4. The medical filter according to claim 3, wherein
the non-fusion region extends along a width direction of the housing orthogonal to a thickness direction of the housing and an axial direction of the inflow port, and
a width of the non-fusion region in the width direction is larger than a width of the filter medium in the width direction.

5. The medical filter according to claim 4, wherein
a width of the outflow region in the width direction is larger than the width of the filter medium in the width direction, and
the filter medium fusion region separates the non-fusion region and the outflow region.

6. The medical filter according to claim 2, wherein
the inflow port is inserted into the inflow region through a proximal portion of the filter medium,
the filter medium fusion region is integrally fused to at least one of two side edge portions of the filter medium between the proximal portion and a distal portion of the filter medium, the proximal portion, and the inflow port,
the non-fusion region includes:
a width direction portion formed between the filter medium fusion region and the peripheral edge fusion region and extending along a width direction of the housing orthogonal to a thickness direction of the housing and an axial direction of the inflow port, and
an axial direction portion extending along the axial direction,
a proximal end of the filter medium is located in the width direction portion,
a side edge of at least one of the side edge portions is located in the axial direction portion, and
the window portion is provided in the housing so as to face the non-fusion region.

7. The medical filter according to claim 6, wherein
a width of the width direction portion in the width direction is larger than a width of the filter medium in the width direction.

8. The medical filter according to claim 7, wherein
the axial direction portion extends toward the outflow port from the distal portion,
the outflow region extends toward the outflow port from the distal portion, and
the filter medium fusion region separates the non-fusion region and the outflow region.
